(19) Europäisches Patentamt
European Patent Office
Office européen des brevets

(11) **EP 3 330 744 A1**

(12) **EUROPEAN PATENT APPLICATION**
published in accordance with Art. 153(4) EPC

(43) Date of publication:
06.06.2018 Bulletin 2018/23

(51) Int Cl.:
*G01T 1/29* (2006.01)     *H05H 13/00* (2006.01)
*H05H 13/04* (2006.01)

(21) Application number: 16830549.8

(22) Date of filing: 27.07.2016

(86) International application number:
PCT/JP2016/071996

(87) International publication number:
WO 2017/018444 (02.02.2017 Gazette 2017/05)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 30.07.2015 JP 2015151323
29.09.2015 JP 2015191754

(71) Applicant: Kaneka Corporation
Osaka-shi, Osaka 530-8288 (JP)

(72) Inventors:
• TATAMI, Atsushi
  Settsu-shi
  Osaka 566-0072 (JP)
• TACHIBANA, Masamitsu
  Settsu-shi
  Osaka 566-0072 (JP)
• MURAKAMI, Mutsuaki
  Settsu-shi
  Osaka 566-0072 (JP)

(74) Representative: Hoffmann Eitle
Patent- und Rechtsanwälte PartmbB
Arabellastraße 30
81925 München (DE)

(54) **GRAPHITE SHEET FOR BEAM SENSOR, BEAM SENSOR ELECTRODE EMPLOYING SAME, AND BEAM SENSOR**

(57) An object of the present invention is to provide a graphite sheet for a beam sensor, which is excellent in yield when subjected to laser working. The present invention is a graphite sheet for a beam sensor characterized in that the graphite sheet has no eyeball-shaped convex portions on a surface of its a-b plane.

[Fig.1]

**Description**

TECHNICAL FIELD

The present invention relates to a graphite sheet for a beam sensor, an electrode for a beam sensor using the sheet, and a beam sensor. The invention preferably relates to an accelerator beam sensor; and a graphite sheet, an electrode and others which are used in the beam sensor.

BACKGROUND ART

An accelerator makes charged particles accelerate to prepare a beam of an aggregate of the particles. An accelerator beam is frequently used in the most-advanced technologies in the fields such as material science, life science, high energy physics, and medical application.

Incidentally, the accelerator beam transmitted is important to be observed at a real time without a breakage of its shape. Desired is an accelerator beam sensor which satisfies no bad effect onto the transmitted beam, a sufficient detection sensitivity, and an endurance permitting continuous use for a long time.

As this accelerator beam sensor, for example, a beam monitoring electrode or a beam monitoring device obtained by using laser working to make a predetermined graphite sheet (carbon graphite thin film) into the form of ribbons is known(Patent Document 1).

As carbon graphite thin films are higher in heat resistance than metals and others, the films can realize endurance against a beam irradiation over a long term. However, the carbon graphite thin film in Patent Document 1 is a thin film produced by a method in Patent Document 2. Specifically, the film is a thin film obtained by carbonizing a polyimide obtained in a thermal curing manner, and then graphitizing the carbonized film while being pressured using a hot isostatic press machine. Such carbon graphite thin film fractures with a high probability when subjected to laser working, and has poor yield. Moreover, the film thickness thereof is not easily made small, and examples have the thickness of about 2.2 $\mu$m. Thus, there is a limit to decrease a beam loss when the accelerator beam transmits through the film. Moreover, after the film is worked into the form of ribbons, a variation in the respective electrical resistances of the ribbons is also large.

PRIOR ART DOCUMENTS

PATENT DOCUMENTS

[Patent Document 1] JP2007-101367
[Patent Document 2] JP2002-308611

SUMMARY OF THE INVENTION

PROBLEMS TO BE SOLVED BY THE INVENTION

Thus, an object of the present invention is to provide a graphite sheet for a beam sensor (preferably graphite sheet for an accelerator beam sensor), which is excellent in yield when subjected to laser working. This graphite sheet is also usable in beam sensors for devices other than accelerators.

In a preferred embodiment, the present invention also has objects such as decreasing a beam loss, improving the endurance of the graphite sheet, and measuring the shape of a radiated beam at real time without affecting the beam substantially.

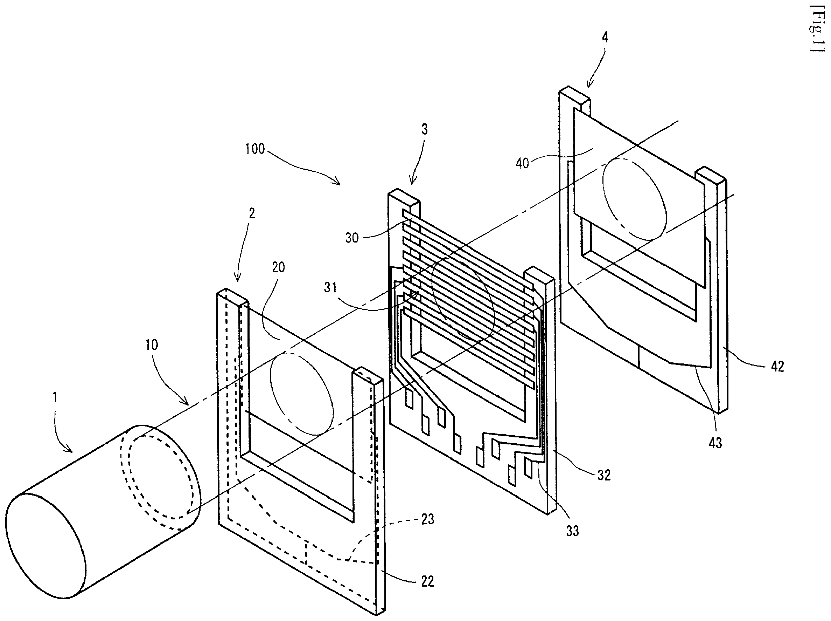

By the way an electrode for a beam sensor of the present invention which is illustrated in Fig. 1 as an example, signals from graphite ribbons corresponding to a central portion of a beam are large while signals from graphite ribbons corresponding to the edge of the beam are small. Accordingly, the dynamic range of an A/D converter which reads signals from the graphite ribbons needs to be made large for precisely understanding the shape of the beam. However, when an input permissible range of the sensor is set not to saturate signals corresponding to the central portion of the beam, signals corresponding to the edge thereof become small so that an accidental error is easily generated. Thus, there is a problem that an accidental error becomes large in the calculation of the centroids.

Considering this point, an object of the present invention in a preferred embodiment is to provide an electrode for a beam sensor enabling a precise measurement of the shape of a beam, and a graphite sheet for a beam sensor used in this electrode.

SOLUTIONS TO THE PROBLEMS

In order to solve the problems, the inventors have made eager investigations to find out that an obtained graphite sheet has no eyeball-shaped convex portions, has small accidental errors of thickness, and is improved in yield when subjected to laser working; and in the case of producing such a graphite sheet that has no eyeball-shaped convex portions and has small accidental errors of thickness, the graphite sheet can be made thinner than conventional graphite sheets and a graphite sheet for a beam sensor (preferably, a graphite sheet for an accelerator beam sensor), which has a small beam loss when used in the beam sensor and a sufficient endurance and detection sensitivity, can be obtained. In this way, the present invention has been achieved.

When one or both of two factors that are the widths of graphite ribbons and intervals therebetween are varied as the need arises, the shape of a beam is more precisely measurable.

In other words, the gists of the present invention are as follows.

[1] A graphite sheet for a beam sensor, having no eyeball-shaped convex portions on a surface of its a-b plane (sheet plane).

[2] A graphite sheet for a beam sensor, having the variation in film thickness of 20% or less.

[3] The graphite sheet according to [1] or [2], which is for an accelerator beam sensor.

[4] The graphite sheet according to [2] or [3], having no eyeball-shaped convex portions on a surface of its a-b plane (sheet plane).

[5] The graphite sheet according to any one of [1] to [4], having a film thickness less than 2.2 $\mu$m.

[6] The graphite sheet according to any one of [1] to [5], wherein the ratio between the resistivity at 5 K and that at 300 K (ratio between the residual resistivities) is 1.2 or more.

[7] The graphite sheet according to any one of [1] to [6], having an electro-conductivity of 16000 S/cm or more.

[8] The graphite sheet according to any one of [1] to [7], which is obtained by using dehydrating agents and one or more selected from tertiary amines to make a film of an aromatic polyimide having a thickness of 100 nm to 7.3 $\mu$m, and sandwiching the resultant aromatic polyimide film between members of one or more species selected from the group consisting of graphite sheets, glassy carbon sheets, graphite plates and glassy carbon plates while pressing to conduct a heat-treatment at a temperature of 2800°C or higher.

[9] An electrode for a beam sensor, wherein the graphite sheet according to any one of [1] to [8] is cut into the form of ribbons, and these graphite ribbons are arranged at regular intervals on the same single plane.

[10] An electrode for a beam sensor, wherein the graphite sheet according to any one of [1] to [8] is cut into the form of ribbons, and these graphite ribbons are arranged on the same single plane in a state that either or both of the widths of the graphite ribbons and intervals between the graphite ribbons are varied.

[11] The electrode for beam sensor according to [9] or [10], wherein the widths of the graphite ribbons are from 100 $\mu$m to 100 mm, the intervals between the graphite ribbons are from 10 $\mu$m to 100 mm, and the lengths of the graphite ribbons are from 10 mm to 800 mm.

[12] A beam sensor, comprising the electrode for a beam sensor according to any one of [9] to [11] and a pair of secondary electron capturing electrodes,
wherein the secondary electron capturing electrodes are arranged in parallel, respectively, to the front surface and the rear surface of the electrode for a beam sensor, and receive secondary electrons emitted from the electrode.

[13] The beam sensor according to [12], wherein a plurality of the electrodes for beam sensors are located to arrange individual electrode planes thereof back and forth while the electrode planes are made parallel to each other, and the graphite ribbons on the individual electrode planes are oriented in directions different from each other.

EFFECTS OF THE INVENTION

The present invention can provide a graphite sheet for a beam sensor (preferably, a graphite sheet for an accelerator beam sensor) excellent in yield when the sheet is laser-worked, and in thickness evenness. Preferably, the graphite sheet can realize thinness, a decrease in beam loss and an improvement of endurance, and measuring the shape of a radiated beam at real time without affecting the beam substantially. A beam sensor (preferably, an accelerator beam sensor) using this graphite sheet is favorably usable both in large accelerators such as a high-intensity proton accelerator, and in ordinary accelerators. The beam sensor is usable in compact accelerators for the public welfare, and is favorably usable in, for example, proton beam therapeutic systems for cancer therapy (therapeutic targets: brain cancer, lung cancer, hepatocellular carcinoma, and prostate cancer; accelerators: cyclotron type and other types); heavy particle beam (for example, carbon ion beam) therapeutic systems (therapy targets: bone, soft tissue sarcoma, and malignant melanoma; accelerators: synchrotron type and other types); boron neutron capture therapeutic (BNCT) systems (therapeutic targets: head and neck cancer, brain tumor, melanoma, mesothelioma, breast cancer, liver cancer, rectal cancer, and anal cancer; beam: a neutron beam (a negative hydrogen ion beam, a proton beam and other beams in the middle stage); accelerators: cyclotron type and other types); and radiopharmaceutical production apparatuses for positron emission tomography (PET) (accelerators: cyclotron type and other types) for PET diagnoses for the purpose of cancer diagnoses (discoveries).

Additionally, the invention makes accidental detection-errors small to heighten the measurement accuracy of the shape of a beam.

BRIEF DESCRIPTION OF THE DRAWINGS

Fig. 1 is a perspective view illustrating an embodiment of the beam sensor of the present invention.

Fig. 2 is a perspective view further illustrating an accelerator in the embodiment of the beam sensor of the invention, which is illustrated in Fig. 1.

Fig. 3 is a schematic plan view illustrating an example of the arrangement state of graphite ribbons in a sensor target used in the beam sensor of the invention.

Fig. 4 is a schematic plan view illustrating another example of the arrangement state of the graphite ribbons in the sensor target used in the beam sensor of the invention.

Fig. 5 is a schematic plan view illustrating still another example of the arrangement state of the graphite ribbons in the sensor target used in the beam sensor of the invention.

Fig. 6 is a schematic plan view illustrating a different example of the arrangement state of the graphite ribbons in the sensor target used in the beam sensor of the invention.

Fig. 7 is a schematic plan view illustrating a still different example of the arrangement state of the graphite ribbons in the sensor target used in the beam sensor of the invention.

Fig. 8 is a schematic plan view illustrating a different example of the arrangement state of the graphite ribbons in the sensor target used in the beam sensor of the invention.

Fig. 9 is a schematic plan view illustrating a still different example of the arrangement state of the graphite ribbons in the sensor target used in the beam sensor of the invention.

Fig. 10 is a schematic plan view illustrating a different example of the arrangement state of the graphite ribbons in the sensor target used in the beam sensor of the invention.

MODE FOR CARRYING OUT THE INVENTION

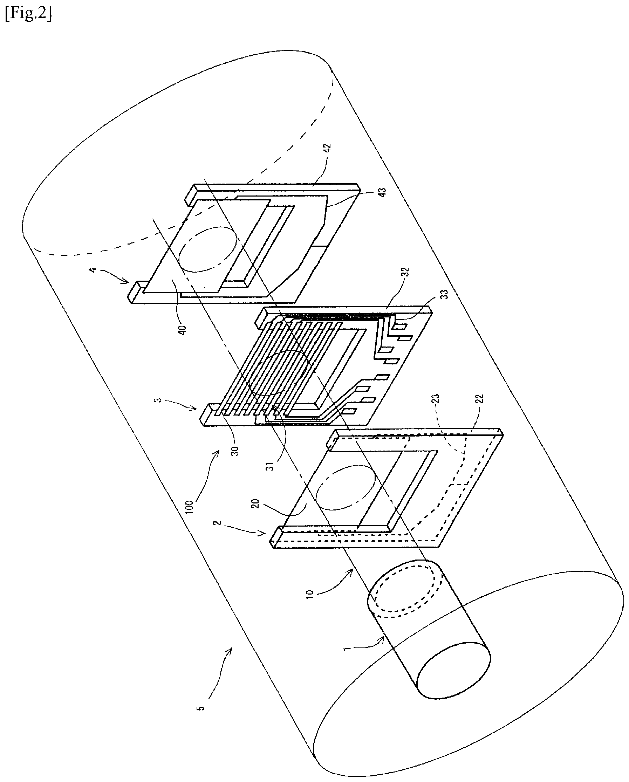

Hereinafter, the present invention will be described in more detail with reference to examples illustrated in the drawings. Fig. 1 is a schematic perspective view illustrating an example of an accelerator beam sensor to which the graphite sheet (multilayered graphene thin film or graphite thin film) of the present invention is applicable. Fig. 2 is a schematic perspective view further illustrating an accelerator 5 in the accelerator beam sensor illustrated in Fig. 1.

In Figs. 1 and 2, the accelerator beam sensor 100 includes, as constituent members thereof, a front-side secondary electron capturing electrode 2, an electrode 3 for an accelerator beam sensor, and a rear-side secondary electron capturing electrode 4.

The electrode 3 for an accelerator beam sensor is used usually in the state of being inserted between the front-side secondary electron capturing electrode 2 and the rear-side secondary electron capturing electrode 4. These are located on an orbit of a beam 10 from a beam halo 1. In the beam sensor of the present invention, both the beam 10 and a profile of the beam halo 1 moiety can be monitored.

In more detail, graphite ribbons 30 are arranged side by side at predetermined intervals (specifically, the shortest distance between adjacent graphite ribbons is made constant, and further the centroid distance between the adjacent graphite ribbons is made constant), and along a horizontal direction in the electrode 3 for an accelerator beam sensor. These graphite ribbons 30 constitute a single sensor target 31 as a whole, and this sensor target 31 is arranged on the beam orbit. In this illustrated example, each of the graphite ribbons 30 and printed wiring lines 33 are drawn in number of eight. However, the number of the ribbons or the wiring lines is not limited to eight, and is appropriately selected in accordance with the beam diameter, the widths of the graphite sheets, intervals between the sheets, and others. The number is set from the range of 2 to 100 (i.e., not less than two and not more than 100), for example.

The graphite ribbons 30 are each fixed in a state capable of electro-conducting to connecting-terminals of the printed wiring lines 33 laid on an insulating frame substrate 32. The terminals are connected to charge integrators not illustrated. A printed wiring line for the ground may be laid on the frame substrate 32.

In the secondary electron capturing electrodes 2 and 4, capturing electrodes 20 and 40 each made of a graphite sheet are each arranged on the beam orbit. These capturing electrodes 20 and 40 are fixed in a state capable of electro-conducting to printed wiring lines 23 and 43 laid, respectively, on insulating frame substrates 22 and 42. Each of the printed wiring lines 23 of the secondary electron capturing electrode 2, and the printed wiring lines 43 of the secondary electron capturing electrode 4 are independently connected to a direct current power source device not illustrated.

When this electrode 3 for an accelerator beam sensor is combined with the secondary electron capturing electrodes 2 and 4 and then a voltage from the direct current power source device is applied to the capturing electrodes 20 and 40 of the secondary electron capturing electrodes 2 and 4 so that the electrodes 20 and 40 have more positive potentials than the sensor target 31 of the accelerator beam sensor electrode 3, the beam profile is measurable. Specifically, charged particles (beam 10) from the beam halo 1 are radiated into the sensor target 31, and then positive charge signals from each of the charge integrator connected to each of the graphite ribbons 30 is detected. In this way, the beam profile of the radiated charged particles beam in the vertical direction can be measured. The positive charge signal from the charge integrator may be amplified through an amplifier to be integrated, and next multiplexed to be displayed on an oscilloscope.

The present invention is characterized by adopting a specific graphite sheet (multilayered graphene thin film) for the graphite ribbons 30 used in the above-mentioned electrode 3 for an accelerator beam sensor. This graphite sheet has features that (1) the sheet has a large area and has no eyeball-shaped convex portions on its surface, and/or is excellent in laser workability owing to being excellent in sheet thickness evenness, and has high yield when producing graphite ribbons; (2) in a preferred embodiment, the sheet does not damage the shape of a beam transmitted through the sheet to enable the measurement of the shape at real time owing to its small thickness and small beam loss, and (3) the sheet can maintain endurance over a longer term than metallic thin films. The capturing electrodes 20 and 40 of the secondary electron capturing electrodes 2 and 4 may make use of a conventional material for secondary electron capturing electrodes, such as an ordinary graphite sheet or a metal thin film. The graphite sheet specified in the present invention is preferably used also for these capturing electrodes 20 and 40. This manner makes it possible to capture secondary electrons effectively while the beam loss is decreased.

The graphite sheet (multilayered graphene thin film) used in the present invention is characterized in that the sheet has no eyeball-shaped convex portions in a surface of its a-b plane (sheet plane or plane in which carbon atoms of graphite are formed in a lattice in the form of hexagonal meshes), and/or that the variation in film thickness is 20% or less. The a-b plane can be referred as the front surface or rear surface of the sheet (any one of two surfaces having the largest area in the three-dimensional shape of the sheet). In conventional graphite sheets, such eyeball-shaped convex portions are present or the variation of film thickness of the sheet is large while in the graphite sheet of the invention these problems are overcome so that the evenness of the surface and the evenness of the film thickness are excellent. The eyeball-shaped convex portions referred to herein typically denote protrusions which are point-symmetric when viewed from above the protrusion, and swell in a mountain form. However, the convex portions are not particularly limited as long as these are convex portions present on the surface of the graphite sheet. For example, the convex portions may be wrinkles formed by the matter that wrinkles produced when a polymeric film is formed are carbonized and graphitized as they are. The convex portions may be wrinkles formed by an uneven shrinkage or expansion caused at the time of the carbonization and graphitization. As far as the convex portions are in the form of eyeballs, the diameter and the height of the eyeballs may be various in value. For example, the eyeball-shaped protrusions may be protrusions that swell in the form of a mountain, and the shape of the protrusions when viewed from above the sheet is a circle or ellipse having a diameter of 1 to 4 mm and the protrusions have a height of 50 $\mu$m to 2 mm; and around the eyeball-shaped convex portions, convex portions may be formed which are each in the form of a concentric circle having a diameter of 1.2 to 8 mm, and a height of 50 $\mu$m to 2 mm when viewed from above the circle (the convex portions may be referred to as depressed portions when viewed from the opposite surface of the graphite sheet). Such concentric circles may be formed in the form of a dual body to a quintuple body around each of the eyeball-shaped convex portions. When convex portions on the sheet plane in the present invention are stripe wrinkles which have narrower dimensions (width) less than 4mm and a dimensional ratio of the length direction to width direction (dimension of length direction / dimension of width direction) is 8 or more at the time of being viewed from above the convex portions, such convex portions are not referred to as eyeball-shaped convex portions. Conventional graphite sheets which have such convex portions (eyeball-shaped convex portions) or large thickness variation may be cracked or cut away in the middle from any one of the convex portions as a starting point in the case of being worked by laser into a predetermined shape, for example, in the case of being cut into the form of ribbons. Thus, the obtained graphite ribbons have a fear of small yield.

The variation of the graphite sheet in film thickness is preferably 20% or less, more preferably 19% or less, even more preferably 18% or less, in particular preferably 17% or less. Even more preferably, the variation is 15% or less, 12% or less, 10% or less, 8% or less, or 5% or less. Even when a graphite sheet has a thickness variation larger than such a value, the graphite sheet with a good laser workability and a small beam loss can be used in the present invention.

When any five points of a graphite sheet are measured about the respective film thicknesses T1 to T5, the arithmetic average obtained therefrom is represented by Tave. Out of the film thicknesses T1 to T5, a film thickness having the largest absolute value of a difference from the value of the film thickness arithmetic average Tave is represented by Tmax. As represented by the following formula (1), the variation V (%) of the graphite sheet in thickness is defined as a value obtained by multiplying the absolute value of the difference between the film thickness Tmax and the arithmetic average Tave of the film thicknesses by 100, and then dividing the resultant value by the arithmetic average Tave of the film thicknesses:

$$V = 100 \times |Tmax - Tave|/Tave \qquad (1)$$

The film thickness of the graphite sheet is preferably made thinner since the film thickness affects a loss of an accelerator beam when the beam penetrates the graphite sheet. The film thickness of the graphite sheet is preferably less than 2.2 $\mu$m, more preferably 1.9 $\mu$m or less, further preferably 1.7 $\mu$m or less, further more preferably 1.5 $\mu$m or less, further preferably 1.3 $\mu$m or less, in particular preferably 1.1 $\mu$m or less. The film thickness is preferably 50 nm or more, more preferably 100 nm or more, further preferably 200 nm or more, further more preferably 300 nm or more, in particular preferably 400 nm or more. As the film thickness becomes smaller while satisfying self-supporting performance, the film doesn't damage the transmission of an accelerator beam so that the loss of the beam can be decreased.

The method for measuring the film thickness may be, for example, a method in a contact manner such as a vernier caliper manner or a probe manner; an optical measuring method using such as a laser displacement meter or a spectroscopic ellipsometer; a measuring method by observing a cross section using an SEM (scanning electron microscope) or a TEM (transmission electron microscope).

The ratio of the graphite sheet between the resistivity at 5 K and that at 300 K (referred to also as the residual resistivity ratio in the document, and means the resistivity at 300 K / the resistivity at 5 K) is preferably 1.2 or more, more preferably 1.6 or more, further preferably 1.8 or more, further more preferably 2.0 or more. The upper limit is, for example, about 10. As the resistivity ratio is higher, the resultant graphite sheet is higher in crystallinity degree; thus, the ratio is an index representing a high quality of the sheet. Such a high-quality graphite sheet has small structural defect to be useful also for decreasing working failures.

The resistivity ratio can be calculated based on the resistivities by an already-known method which is not particularly limited, such as the van der Pauw method or an ordinary four-terminal method. The measurement at 5 K may be carried out in the state that the sample is cooled by an already-known method using such as liquid helium or a helium circulating apparatus.

The electro-conductivity of the graphite sheet is, for example, 16000 S/cm or more, preferably 17500 S/cm or more, more preferably 18500 S/cm or more, further preferably 19500 S/cm or more. The electro-conductivity is also an index of a high quality of the graphite sheet. The electro-conductivity may be, for example, 24000 S/cm or less, or 23000 S/cm or less.

The variation of the graphite sheet in electro-conductivity is preferably 20% or less, more preferably 15% or less, further preferably 10% or less, in particular preferably 5% or less.

The variation in electro-conductivity is defined as a value obtained by measuring the respective electro-conductivities S of plural points of the graphite sheet, obtaining the arithmetic average Save of the electro-conductivities and the electro-conductivity Smax having the largest absolute value of a difference from this Save value, and then calculating in accordance with the following expression:

$$\text{Variation (\%) in the electro-conductivity} = 100 \times |Smax - Save|/Save$$

The electro-conductivity can be calculated by measuring the electrical resistance by an already-known method such as the van der Pauw method or an ordinary four-terminal method, and then using the dimension and the thickness of the sample.

The area of the graphite sheet is not particularly limited as far as the area permits graphite ribbons, which is used in an electrode for an accelerator beam sensor, for example, to be cut out from the sheet. The area is, for example, 1 x 1 cm$^2$ or more, preferably 2 x 2 cm$^2$ or more, more preferably 3 x 3 cm$^2$ or more, further preferably 5 x 5 cm$^2$ or more, further more preferably 10 x 10 cm$^2$ or more, in particular preferably 20 x 20 cm$^2$ or more, most preferably 30 x 30 cm$^2$ or more. The graphite sheet may have, for example, a size of 10 x 26 cm, a size of 15 x 35 cm, or a size of 20 x 40 cm. When the graphite sheet is a graphite sheet having such a large area, all of the graphite ribbons 30, which constitute the set of the sensor targets 31, can be cut out from the single sheet. Accordingly, the variation of the ribbons in film thickness, inside the sensor target 31, becomes even so that the variation thereof in electrical resistance can also become even.

The upper limit of the area of the graphite sheet is not particularly limited as far as the graphite sheet can be produced. The area is, for example, an area of 80 x 80 cm$^2$ or less, preferably an area of 70 x 70 cm$^2$ or less.

The heat resistant temperature (sublimation) of the graphite sheet is, for example, 3000°C or higher, or 3100°C or higher. The graphite sheet having such a high heat resistant temperature has a sufficient heat resistance and endurance even when irradiated with an accelerator beam over a long term.

The graphite sheet used in the present invention can be produced, for example, by carbonizing and graphitizing a specific polymeric film as a raw material by a specific method. For this polymeric film as a raw material, an aromatic polyimide is usable which is produced from an acid dianhydride (particularly, an aromatic acid dianhydride) and a diamine (particularly, an aromatic diamine) via a polyamic acid.

Examples of the acid dianhydride used to synthesize the aromatic polyimide include pyromellic dianhydride (PMDA), 2,3,6,7-naphthalenetetracarboxylic dianhydride, 3,3',4,4'-biphenyltetracarboxylic dianhydride, 1,2,5,6-naphthalenetetracarboxylic dianhydride, 2,2',3,3'-biphenyltetracarboxylic dianhydride, 3,3' ,4,4' -benzophenonetetracarboxylic dianhydride, 2,2-bis(3,4-dicarboxyphenyl)propane dianhydride, 3,4,9,10-perylenetetracarboxylic dianhydride, bis(3,4-dicarboxyphenyl)propane dianhydride, 1,1-bis(2,3-dicarboxyphenyl)ethane dianhydride, 1,1-bis(3,4-dicarboxyphenyl)ethane dianhydride, bis(2,3-dicarboxypenyl)methane dianhydride, bis(3,4-dicarboxyphenyl)ethane dianhydride, oxydiphthalic dianhydride, bis(3,4-dicarboxyphenyl)sulfonic dianhydride, p-phenylenebis(trimellitic acid monoester anhydride), ethylene bis(trimellitic acid monoester anhydride) and bisphenol A bis(trimellitic acid monoester anhydride) and analogues thereof, and these can be used solely or a mixture of any desired ratio. For the view point that the polyimide film having a polymer architecture with a very rigid structure has higher orientation so that a graphite excellent in crystallinity is easily obtained, and from the view point of availability, pyromellitic anhydride, and 3,3' ,4,4' -biphenyltetracarboxylic dianhydride are particularly preferred.

Examples of the diamine used to synthesize the aromatic polyimide include 4,4'-diaminodiphenyl ether (ODA), p-phenylenediamine (PDA), 4,4'-diaminodiphenylpropane, 4,4' -diaminodiphenylmethane, benzidine, 3,3' -dichlorobenzidine, 4,4' -diaminodiphenylsulfide, 3,3' -diaminodiphenylsulfone, 4,4' -diaminodiphenylsulfone, 3,3'-diaminodiphenyl ether, 3,4' -diaminodiphenyl ether, 1,5-diaminonaphthalene, 4,4'-diaminodiphenyldiethylsilane, 4,4' -diaminodiphenylsilane, 4,4' -diaminodiphenylethylphosphine oxide, 4,4' -diaminodiphenyl-N-methylamine, 4,4' -diaminodiphenyl-N-phenylamine, 1,4-diaminobenzene(p-phenylenediamine), 1,3-diaminobenzene, and 1,2-diaminobenzene and analogues thereof, and these can be used solely or a mixture of any desired ratio. From the view point that an orientation of a polyimide film becomes high and a graphite excellent in crystallinity is easily obtained, and from the view point of availability, 4,4' -diaminodiphenyl ether (ODA) or p-phenylenediamine (PDA) is preferably used.

The polyamic acid is recommendable to be imidized by a chemical curing method using a dehydrating agent typified by acid anhydride such as acetic anhydride, or tertiary amines such as picoline, quinoline, isoquinoline, and pyridine as an imidization promoting agent. The polyimidization according to the chemical curing method makes physical properties of the resultant graphite better than a thermal curing method in which polyamic acid is imidized by heating.

A method for producing a polyimide film by the chemical curing is, for example, as follows: To a solution of the polyamic acid in an organic solvent, a dehydrating agent in an amount more than the chemical stoichiometric amount thereof and an imidization promoter in a catalytic amount are initially added. The resultant is cast or applied onto a supporting substrate such as an aluminum foil, a polymeric film made of, PET and the like, or a support such as a drum or an endless belt to give a film. The resultant is heated to dry the organic solvent to obtain a film having self-supporting performance. Next, this film is imidized while heating and being dried. In this way, a polyimide film is obtained. The temperature in the heating ranges preferably from 150 to 550°C.

Without adding the above-mentioned imidization promoter, the polyamic acid may be simply heated to be imidized, thereby yielding a polyimide film (thermal curing). The heating temperature in this case also ranges preferably from 150 to 550°C.

The thickness of a I polymeric film (aromatic polyimide film) as a raw material ranges preferably from 100 nm to 7.3 $\mu$m (i.e., not less than 100 nm and not more than 7.3 $\mu$m) to obtain a graphite sheet (graphite film) satisfying a film thickness range in the present invention. The thickness ranges more preferably from 200 nm to 5 $\mu$m (i.e., not less than 200 nm and not more than 5 $\mu$m), more preferably from 300 nm to 4 $\mu$m (i.e., not less than 300 nm and not more than 4 $\mu$m). The reason is as follows: the thickness of the finally obtained graphite film is often from about 60 to 30% (i.e., not more than 60% and not less than 30%) of the thickness of the polymeric film as a raw material when the thickness of the polymeric film as a raw material is 1 $\mu$m or more, and the final thickness is often from about 50 to 20% (i.e., not more than 50% and not less than 20%) of the raw material film thickness. As the thickness of the used raw material polymeric film is smaller, physical properties of the resultant graphite can be made better.

The polymeric film as a raw material obtained as described above is heated in an inert gas or a vacuum to be carbonized. The inert gas is preferably nitrogen, argon, or a mixed gas of nitrogen and argon. The carbonization is performed at a temperature ranging from about 800 to 1800°C (i.e., not lower than 800°C and not higher than 1800°C). For example, the method is preferably adopted in which the polymeric film is heated at a heating rate of 10°C/minute up to about 800 to 1800°C, and is kept as it is while retaining the temperature for a period from about 10 minutes, for example, 5 minutes to 5 hours (not shorter than 5 minutes and not longer than 5 hours), preferably from 10 minutes to 2 hours (not shorter than 10 minutes and not longer than 2 hours). The heating rate is not particularly limited, and is preferably 0.5°C/minute or more from the viewpoint of an improvement of the productivity. Moreover, this rate is preferably 100°C/minute or less to sufficiently carbonize the film. In general, the rate is preferably between 1 and 50°C/minute (not less than 1°C/minute and not more than 50°C/minute). The heating manner in the carbonization is not particularly limited, and is preferably a manner using a resistance heating type heater such as a graphite heater, or a manner using infrared radiation.

The carbonized film, which has been carbonized by the above-mentioned method, is set in a graphitizing furnace to be graphitized. In order to create a high temperature of 2200°C or higher, which is necessary for the graphitization, an electric current is usually flowed into a graphite heater, and the resultant Joule heat is used to heat the film. The graphitization is performed in an inert gas, and argon is most suitable as the inert gas. More preferably, a small amount of helium is added to argon.

As the heating temperature is higher, a graphite sheet having a higher electro-conductivity is easily obtained. A polymeric film having, particularly, a thickness of 7.3 $\mu$m or less is easily converted to graphite even at a relatively low temperature. Thus, a heating temperature necessary for obtaining the graphite sheet of the present invention is relatively low, and is 2200°C or higher. The fact that the graphitization is possible at such a relatively low temperature is advantageous since costs can be decreased by the simplification of the graphitizing furnace or electric power reduction. Of course, when the graphite is desired to have higher quality, higher temperature in the graphitization is better. The graphitization is preferably performed by heating at a temperature of 2600°C or higher, more preferably 2800 °C or higher, most preferably 3000°C or higher. The upper limit of the thermal treatment temperature may be, for example, about 3500°C. The heating rate up to the thermal treatment temperature in the graphitization is not particularly limited, and is, for example, from 0.5 to 100°C/minute (i.e., not less than 0.5°C/minute and not more than 100°C/minute), preferably from 1 to 50°C/minute (not less than 1°C/minute and not more than 50°C/minute). The retention period at the thermal treatment temperature in the graphitization is, for example, from 10 minutes to 1 hour (not shorter than 10 minutes and not longer than 1 hour).

The graphite sheet of the present invention may be produced, in a graphitizing step as described above in which carbonized films are thermally treated to be graphitized, by laminating the carbonized films onto each other; sandwiching the laminate between gaskets made of graphite, graphite sheets obtained by firing polyimide films, glassy carbon sheets, or other auxiliary sheets; sandwiching the resultant further between isotropic graphite plates such as a CIP (cold isotropic press) material, glassy carbon plates such as glassy carbon, or other auxiliary plates; and treating the resultant workpiece thermally while pressing the workpiece.

When the laminate is sandwiched between the auxiliary sheets and/or the auxiliary plates, and then subjected to the thermal pressing treatment to be graphitized (preferably sandwiched between the auxiliary sheets, sandwiched further between the auxiliary plates, and then subjected to the thermal pressing treatment to be graphitized), no eyeball-shaped convex portions are formed, and the film thickness variation of the sheet can be made small. As a result, a graphite sheet can be obtained which is good in cutting workability by a laser and which can give a worked product good in electrical resistance variation.

A carbonized film may be produced and then sandwiched between the auxiliary sheets and/or the auxiliary plates. However, at the stage of a polymeric film as a raw material, which has not yet been carbonized, this polymeric film may be sandwiched between the sheets and/or the plates. Plural product-sets in each of which the carbonized film or the polymeric film as a raw material is sandwiched between the auxiliary sheets and/or the auxiliary plates may be laminated onto each other, and then the resultant is set into a firing furnace. The thermal pressing treatment of the products, in each of which the carbonized film or the raw material polymeric film is sandwiched between the auxiliary sheets and/or the auxiliary plates, may be performed at only the graphitizing stage, or at both of the carbonization and the graphitization stages. A graphite sheet obtained by graphitizing a carbonized film once may be sandwiched between the auxiliary sheets and/or the auxiliary plates, and then pressed while heated again to the graphitizing temperature (2200°C or higher).

When the product-sets, in each of which the carbonized film, the polymeric film as a raw material or the graphite sheet is sandwiched between the auxiliary sheets and/or the auxiliary plates, are laminated onto each other and then set in a firing furnace, the number of the laminated sets is, for example, 2 or more, preferably 5 or more, more preferably 10 or more (in other words, the number of the carbonized films, the polymeric film as a raw material or the graphite sheets that are heated at a time is, for example, 2 or more, preferably 5 or more, further preferably 10 or more). When a plurality of carbonized films, polymeric films as raw materials or graphite sheets are heated at a time in such a way or are heated and pressed, one, two, or three or more auxiliary sheets and/or auxiliary plates may be inserted into between any two of the carbonized films, polymeric films as raw materials or graphite sheets. The number of the inserted sheet(s) and/or plate(s) may be appropriately adjusted. When a plurality of the carbonized films, polymeric films as raw materials or graphite sheets are fired at a time, the films (sheets), and the auxiliary sheets and/or the auxiliary plates are preferably put vertically onto each other to make their edges consistent each other without shifting these members out of position. If these members are shifted out of position, an even load is not applied to the laminate particularly at the time of the pressing thereof, so that a graphite sheet having many wrinkles or much strain is obtained.

The pressing pressure in the heating is preferably not less than 0.1 kgf/cm$^2$ and not more than 200 kgf/cm$^2$, more preferably not less than 0.2 kgf/cm$^2$ and not more than 100 kgf/cm$^2$, even more preferably not less than 0.3 kgf/cm$^2$

and not more than 50 kgf/cm$^2$.

When the above-mentioned graphite sheet is used for an electrode for an accelerator beam sensor, for example, the graphite sheet may be fixed onto a frame substrate and sensor target terminals (terminals of the part which lies at a tip portion of printed wiring lines on the frame substrate and contacts with the graphite) and then cut into a desired shape, or a product obtained by cutting the graphite sheet beforehand into a desired shape may be fixed onto the terminals of the sensor target.

The shape of the graphite sheet may be any shape as far as the shape can connect the terminals of the sensor target to each other. The shape is, for example, a square, a rectangle, or a bow shape, and is preferably a ribbon shape.

The widths of the graphite ribbons are each preferably from 100 $\mu$m to 100 mm (i.e., not less than 100 $\mu$m and not more than 100 mm), more preferably from 200 $\mu$m to 50 mm (i.e., not less than 200 $\mu$m and not more than 50 mm), further preferably from 500 $\mu$m to 10 mm (i.e., not less than 500 $\mu$m and not more than 10 mm), further more preferably from 500 $\mu$m to 2 mm (i.e., not less than 500 $\mu$m and not more than 2 mm) from the viewpoint of a desired number of the graphite ribbons (desired number of detecting-positions), the self-supporting performance of the ribbons that permits the ribbons to be fixed between the terminals of the sensor target, the laser workability of the ribbons, and other factors.

The intervals between the graphite ribbons are each preferably from 10 $\mu$m to 100 mm (i.e., not less than 10 $\mu$m and not more than 100 mm), more preferably from 50 $\mu$m to 50 mm (i.e., not less than 50 $\mu$m and not more than 50 mm), further preferably from 100 $\mu$m to 10 mm (i.e., not less than 100 $\mu$m and not more than 10 mm), further more preferably from 200 $\mu$m to 2 mm (i.e., not less than 200 $\mu$m and not more than 2 mm) to restrain the interference of signals between adjacent graphite ribbons.

The lengths of the graphite ribbons are each preferably from 10 to 800 mm (i.e., not less than 10 mm and not more than 800 mm), more preferably from 20 to 700 mm (i.e., not less than 20 mm and not more than 700 mm), further preferably from 30 to 500 mm (i.e., not less than 30 mm and not more than 500 mm), further more preferably from 40 to 400 mm (i.e., 40 mm or more, and 400 mm or less) from the viewpoint of the laser workability and the self-supporting performance of the ribbons, and the effective diameter of a space for beam-travelling.

A method for producing the graphite ribbons is as follows:
An appropriate fixing means such as an adhesive is used to fix a graphite sheet onto terminals of printed wiring lines of a frame substrate. The graphite sheet may be fixed to block the orbit of an accelerator beam in the frame substrate. In this case, the fixation may be performed while tension is applied to the edge of the graphite sheet.

Next, the graphite sheet may be worked into the form of ribbons by irradiating with a laser to form the sensor target.

In the meantime, a graphite sheet is worked into the form of ribbons using a laser, and the resultant graphite ribbons may be fixed using an adhesive to individual terminals of printed wiring lines of a frame substrate. The adhesive is preferably an electro-conductive adhesive.

The laser is preferably a known working laser such as an ultraviolet laser, a carbon dioxide gas laser, a YAG laser, a YVO$_4$ laser, a fiber laser, or an excimer laser.

According to the above description, the electrode for a beam sensor (preferably the electrode for an accelerator beam sensor) of the present invention is characterized in that the above graphite sheet is cut in the form of ribbons, and these graphite ribbons are arranged on the same single plane. The monitoring electrode (beam sensor electrode) of the invention decreases beam loss so that the shape of an accelerator beam and the radiation state thereof are measurable, as they are, at real time.

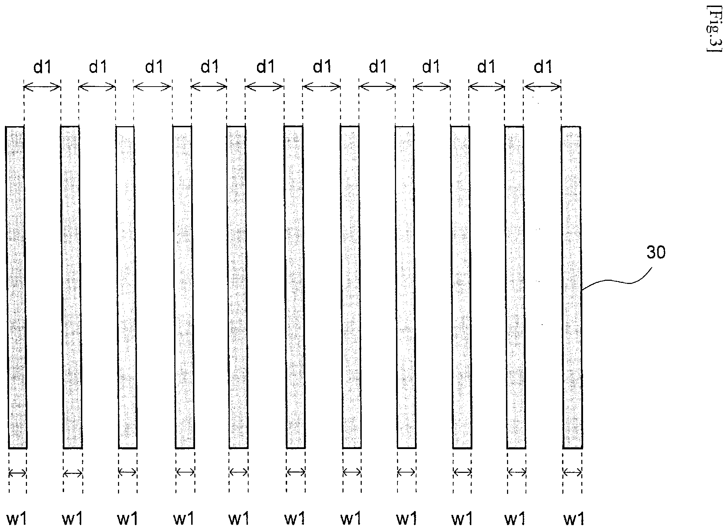

In this monitoring electrode, the number, the width, the length, and the film thickness of the graphite ribbons; and intervals between the graphite ribbons can be adjusted appropriately in accordance with an accelerator beam to be used. For example, Fig. 3 is a partial enlarged schematic plan view illustrating a state that graphite ribbons are arranged at predetermined intervals in Figs. 1 and 2. In this example in Figs. 1, 2 and 3, graphite ribbons 30 are arranged on the same single plane to have the same widths w1 and the same intervals d1. Any sensor target usually takes such a configuration from the viewpoint of the production efficiency.

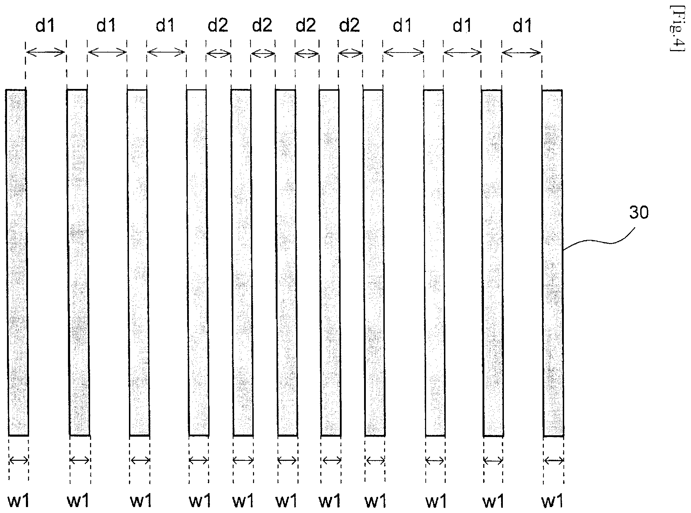

The graphite ribbons may be arranged into various forms in which the graphite ribbons are not arranged at predetermined intervals. The widths of the graphite ribbons may be appropriately varied. Figs. 4 to 10 are each a partial enlarged schematic plan view illustrating a sensor target in which either or both of two factors that are the widths of graphite ribbons and intervals therebetween are appropriately varied, and the ribbons are arranged. A specific form of the graphite ribbons in each of these figures, and advantages thereof are described. The wording "the widths of graphite ribbons are varied" means that the width of at least one of the graphite ribbons is different from the respective widths of the other graphite ribbons. The wording "intervals between graphite ribbons are varied" means that the interval between at least one pair of the graphite ribbons is different from respective intervals between other pairs thereof.

Fig. 4 illustrates an example in which graphite ribbons 30 having the same widths w1 are arranged on the same single plane to have different intervals d1 and intervals d2. The intervals d2 in a central portion of the sensor target are narrower than the intervals d1 in the other portion. Such an arrangement makes it possible to measure the shape of a beam precisely at the portion of narrower interval d2.

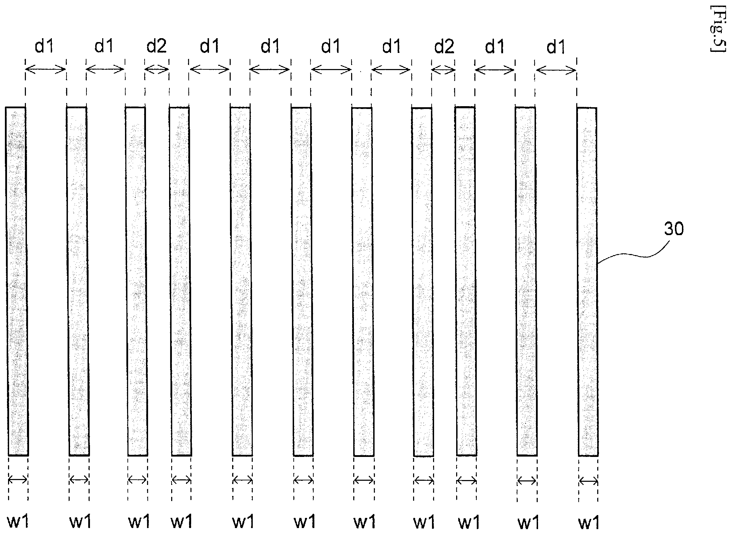

In the same manner as in Fig. 4, graphite ribbons having the same widths w1 are arranged on the same single plane to have different intervals d1 and intervals d2 in Fig. 5. However, in the example in Fig. 5, at two sites of the sensor target that are equally distant from the center, one or more of the intervals d2 (one in the illustrated example) for each of the sites is/are narrower than the intervals d1 at the other sites. The two sites correspond roughly to the circumferential edge of the light bundle of a beam. The beam tends to be largely changed in beam intensity merely when a spot from the position of the center of the light bundle is slightly shifted. This example is effective for heightening measurement accuracy at such a spot.

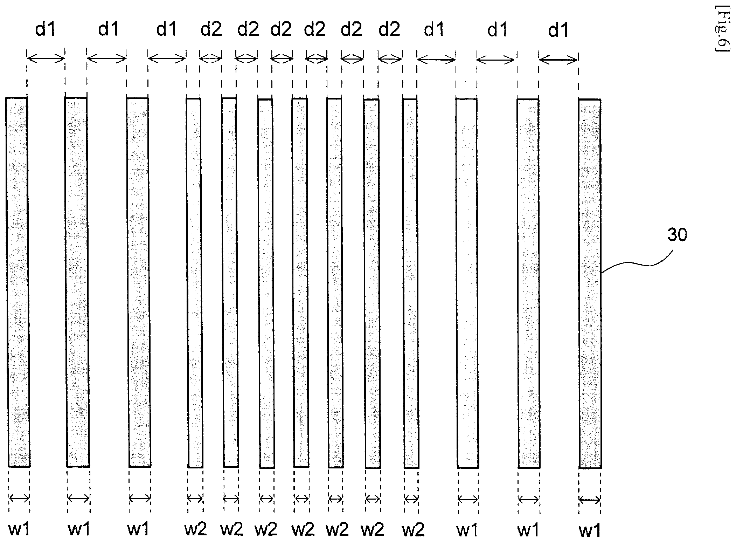

Fig. 6 illustrates an example in which graphite ribbons 30 having different widths w1 and widths w2 (w1 > w2) are arranged on the same single plane to have different intervals d1 and intervals d2 (d1 > d2). The widths w2 in a central portion of the sensor target are narrower than the widths w1 in the other portion. Moreover, the intervals d2 in the central portion are narrower than the intervals d1 in the other portion. Also in this example, the measurement accuracy of the shape of a beam in the central portion can be heightened.

The beam-receiving area of the graphite ribbons positioned in the central portion is small while that of the graphite ribbons positioned near ends of the sensor target is large. Thus, when the shape of an ordinary beam, which has a large intensity near the center thereof, is measured, a difference in intensity between signals detected from the individual graphite ribbons becomes relatively small. This matter makes it possible to set the dynamic range of a detecting device into a small value. Consequently, even a simple detecting device can detect signals with a small accidental error as a whole.

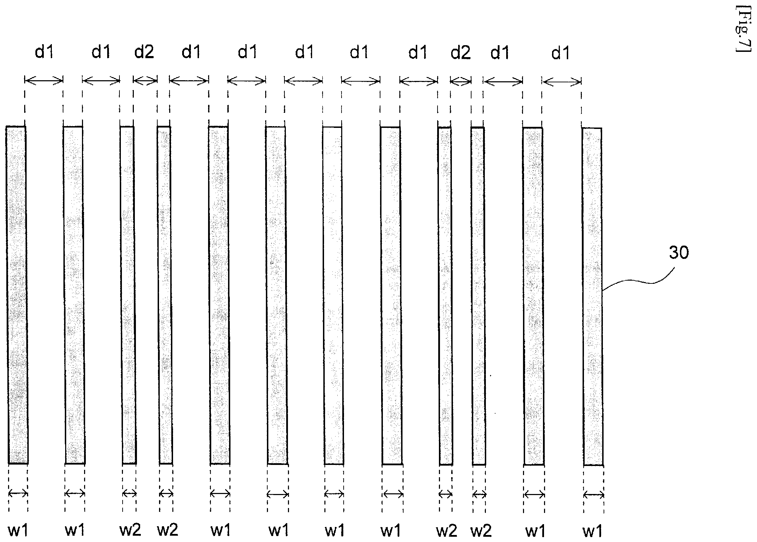

Fig. 7 illustrates an example in which graphite ribbons 30 are arranged on the same single plane to have different widths w1 and widths w2 (w1 > w2) and different intervals d1 and intervals d2 (d1 > d2), a plurality of the narrower intervals d2 are arranged. In the illustrated example, at two sites of the sensor target that are equally distant from the center, one or more (two in the illustrated example) of the graphite ribbons that (each) has/have the narrower width w2 at each of the sites is/are arranged with one or more (one in the illustrated example) of the narrower intervals d2 at each of the sites. At the sites, the shape of a beam can be precisely measured.

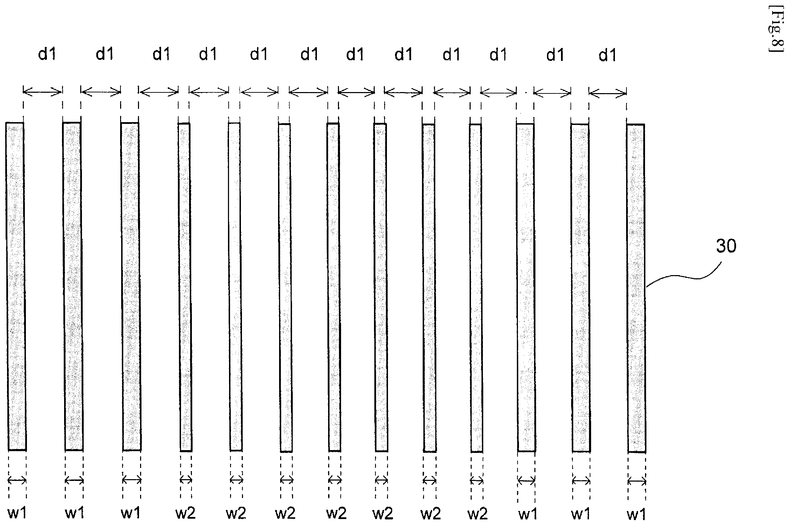

Fig. 8 illustrates an example in which graphite ribbons 30 having different widths w1 and widths w2 (w1 > w2) are arranged on the same single plane to have the same intervals d1. In the illustrated example, the widths w2 of the graphite ribbons positioned in a central portion of the sensor target is narrower than those w1 of the graphite ribbons positioned in the other portion. When the shape of an ordinary beam, which has a large intensity near the center thereof, is measured, a difference in intensity between signals detected from the individual graphite ribbons becomes relatively small. This matter makes it possible to set the dynamic range of a detecting device into a small value. Consequently, even a simple detecting device can detect signals with a small accidental error as a whole.

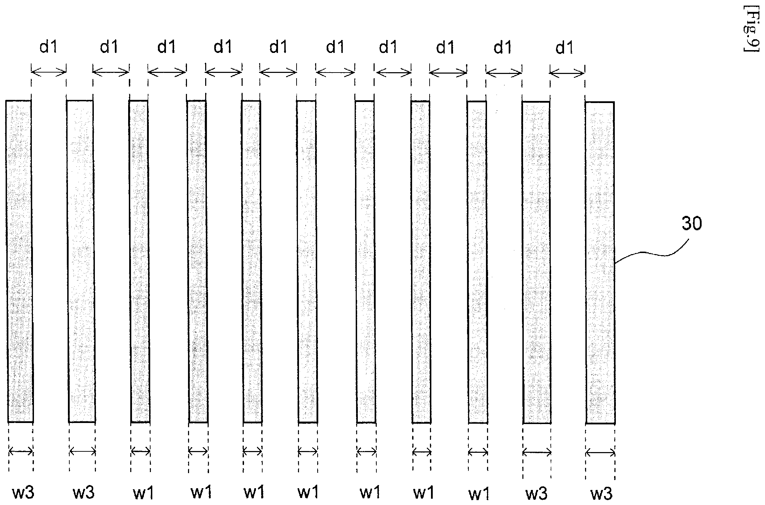

Fig. 9 illustrates an example in which graphite ribbons 30 having different widths w1 and widths w3 (w3 > w1) are arranged on the same single plane to have the same intervals d1. In the illustrated example, one or more (two in the illustrated example) of the graphite ribbons that (each) has/have a larger width than the widths of the graphite ribbons positioned in a central portion of the sensor target are arranged with the regular intervals d1 at one or more peripheral portions to the end of the sensor (at two peripheral portions to the end of the sensor in the illustrated example). In a portion of the sensor target where a low-intensity beam is irradiated, an electric current signal becomes high in accordance with the ribbon widths. Thus, the use of the graphite ribbons with the broad widths w3 improves the measurement sensitivity of the beam shape. Moreover, when the shape of an ordinary beam, which has a large intensity near the center thereof, is measured, a difference in intensity between signals detected from the individual graphite ribbons becomes relatively small. This matter makes it possible to set the dynamic range of a detecting device into a small value. Consequently, even a simple detecting device can detect signals with a small accidental error as a whole.

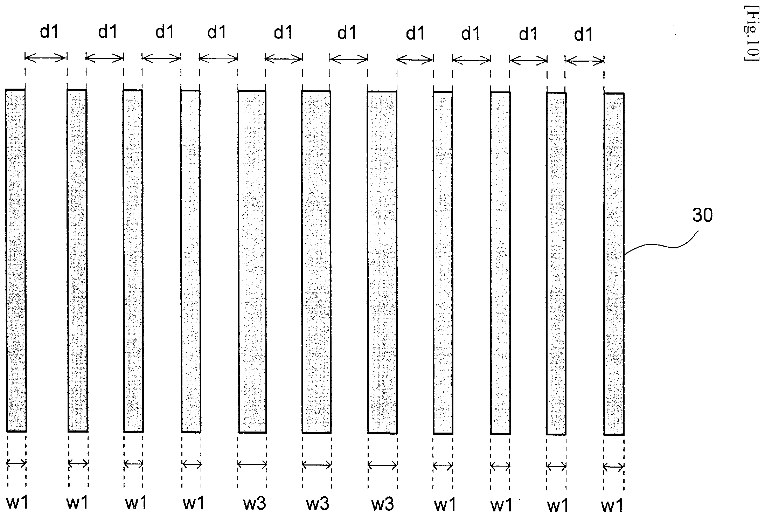

Fig. 10 illustrates another example in which graphite ribbons 30 having different widths w1 and widths w3 (w3 > w1) are arranged on the same single plane to have the same intervals d1. In the illustrated example, one or more (three in the illustrated example) of the graphite ribbons that (each) has/have a broader width than the widths of the graphite ribbons that are used in peripheral portions to the end of the sensor target are arranged, with regular intervals d1, at a central portion of the sensor target. The use of the graphite ribbons having the broad widths w3 in a portion of the sensor target where a high-intensity beam is irradiated contributes to an improvement of the graphite ribbons in endurance.

As illustrated in Figs. 3 to 10, the present invention also includes embodiments in each of which a plurality of graphite ribbons are arranged to the same single plane to make either or both of width(s) and interval(s) of graphite ribbons (the interval means the shortest distance or the centroid distance; the shortest distance is illustrated in the examples) different, in accordance with a beam to be used or the purpose.

In such different examples, the widths of graphite ribbons may be the same or different. When the widths of graphite ribbons are different, a plurality of graphite ribbons having different width from the others may be used. In the case of using the graphite ribbons with different widths, the widths thereof are classified relatively into narrower widths and broader widths. The graphite ribbons with narrower widths may be used in a portion of the sensor target where the measurement accuracy of the beam shape is to be heightened. The graphite ribbons with broader widths may be used in a portion of the sensor target where the intensity of the beam is weak from the viewpoint of an improvement of the beam sensor in sensitivity, or used in a portion of the sensor target where the intensity of the beam is strong from the viewpoint of an improvement of the endurance of the graphite ribbons.

The respective intervals between the graphite ribbons may be the same or different. When the intervals of graphite ribbons are different, a plurality of intervals different from the others may exist. When different intervals between the graphite ribbons exist, the intervals thereof are classified relatively into narrower intervals and broader intervals. The narrower intervals may be arranged in a portion of the sensor target where the measurement accuracy is to be heightened.

In the accelerator beam sensor of the present invention which is illustrated in Fig. 1 as an example, the number of beam particles transmitted through the graphite ribbons which constitute the sensor target, is varied in accordance with the individual positions of the graphite ribbons. In other words, when all the graphite ribbons are equal in width to each other (for example, in Fig. 3), the beam which the graphite ribbons receive is varied in accordance with respective positions where the graphite ribbons are arranged. A beam is emitted from an accelerator, and the number of particles of the beam transmitted through the graphite ribbons positioned in a central portion of the beam light bundle (in the case of a circular beam, the central portion is a center portion of the circle) is larger than that through the graphite ribbons positioned at a peripheral edge portion of the light bundle (in the case of a circular beam, the circumferential edge portion is a circumferential portion of the circle).

Accordingly, a difference is generated in detected beam intensity between the graphite ribbons located at the central portion of the light bundle (such as a center portion of a circular-beam), and those located at the peripheral edge portion of the light bundle (such as a circumferential portion of a circular-beam). This matter may cause an accidental detection-error. An effective method for making such an accidental error small is, for example, to decrease the widths of the graphite ribbons located in the central portion of the light bundle (such as a center portion of a circle) (for example, in Figs. 6 and 8), or to increase the widths of the graphite ribbons located at the peripheral edge portion of the light bundle (such as a circumferential portion) (for example, in Fig. 9). In such a case, the respective widths of the graphite ribbons are different from each other in accordance with positions where the ribbons are located, so that the graphite ribbons are not arranged at predetermined intervals. However, no especial problem is caused. In the case of desiring to make detecting-sites of the beam denser at a partial position of the beam, it is effective to make intervals between the graphite ribbons narrower at this position (for example, in Figs. 4, 5, 6 and 7).

In a central portion of the sensor target at which a central portion of a beam is measured, the beam intensity tends to become strong. By making the graphite ribbon width broad in the central portion of the sensor target, the sensor target can be improved in endurance.

In the above-mentioned examples, the intervals between the graphite ribbons are defined as the respective distances between ends of adjacent graphite ribbons, these ends being ends close to each other. However, the intervals may be defined as the respective centroid distances between the adjacent graphite ribbons.

The raw material of any member other than the graphite sheet may be an appropriate known raw material adopted for the use. For example, the frame substrates 22, 32 and 42 are preferably made of a raw material with electrically insulating property, radial ray resistance, and a low gas-emitting property in a vacuum. Such a material may be a ceramic material. For example, alumina, and silicon nitride are preferred, considering the strength and the thermal conduction thereof.

The present invention includes: an accelerator beam sensor comprising the above-defined electrode for an accelerator beam sensor; and a pair of secondary electron capturing electrodes that are arranged in parallel, respectively, to the front surface and the rear surface of the sensor electrode, and receive secondary electrons emitted from the sensor electrode.

The interval between the electrode 3 for an accelerator beam sensor and the secondary electron capturing electrode 2 (the interval between the graphite thin films) may be appropriately set, considering the gas-discharging property of the vacuum. When the area of the frame substrate 22 is, for example, 10000 mm$^2$ or less, the interval is preferably from 2 to 10 mm (i.e., not less than 2 mm and not more than 10 mm), more preferably from 3 to 10 mm (i.e., not less than 3 mm and not more than 10 mm). When the area of the frame substrate 22 is more than this value 10000 mm$^2$, the upper limit of the interval may be set to about 15 mm. The same is applicable to the interval between the accelerator beam sensor electrode 3 and the secondary electron capturing electrode 4.

Individual leading-out terminals of the printed wiring lines 23 of the secondary electron capturing electrode 2 are connected to an anode terminal of a DC power source device for voltage-application. When the emitted amount of the secondary electrons is large, a capacitor may be inserted into this anode terminal.

In each of the illustrated examples, an electrode for an accelerator beam sensor that has a sensor target composed of graphite ribbons arranged side by side in the horizontal direction is illustrated. However, the direction along which the graphite ribbons are arranged side by side may be set to be matched with a profile of a beam to be measured. The graphite ribbons do not necessarily need to be fixed in parallel to sides of the frame substrate, and may be fixed in a direction oblique to the sides. Furthermore, profiles of beams in different directions may be simultaneously measured by using, together, monitoring electrodes having plural (for example, two) sensor targets in which graphite ribbons are arranged side by side in different directions (for example, in directions orthogonal to each other).

In other words, plural electrodes for accelerator beam sensors as described above may be located to arrange individual electrode planes thereof back and forth while making the individual electrode planes parallel to each other, so that graphite ribbons on the individual electrode planes are oriented in directions different from each other. In this case, besides the front-side secondary electron capturing electrode and the rear-side secondary electron capturing electrode, a secondary electron capturing electrode may be further located between the accelerator beam sensor electrodes.

The energy of a charged particle beam the profile of which is measurable through a beam sensor as described above is 1 keV or more in the case of lightweight charged particles such as electrons. Even in the case of heavier charged particles, the energy is, for example, 100 keV or more. Considering beam loss, the energy is desirably made as high as possible. The energy per nucleon is desirably 1 MeV or more, 500 MeV or more, or 1 GeV or more, and may be 10 GeV or more, 30 GeV or more, or 100 GeV or more.

The present invention is applicable not only to accelerator beam sensors but also to other various beam sensors. Examples of such articles include electrodes for beam intensity monitors (the number of secondary electrons is counted through their capturing electrodes), beam monitors for monitoring a loss beam shifted out from the center of the course of a beam, and other beam monitors. An apparatus for generating the beam is not particularly limited; thus, for example, the invention is applicable to a beam sensor for a beam emitted from a nuclear reactor.

The present application claims priorities based on Japanese Patent Application No. 2015-151323 filed on July 30, 2015, and Japanese Patent Application No. 2015-191754 filed on September 29, 2015, and the entire contents of descriptions of the Japanese Patent Application No. 2015-151323 filed on July 30, 2015, and Japanese Patent Application No. 2015-191754 filed on September 29, 2015 are incorporated into the present application for reference.

EXAMPLES

Hereinafter, the present invention will be more specifically described by using examples thereof. However, the invention is not limited by the following examples. Of course, the examples may be appropriately modified and carried out as far as the modified examples conform to subject matters of the invention that have been described above or will be described below. These modified examples are included in the technical scope of the invention.

[Measurement of Residual Resistivity Ratio]

From a partial area of a produced graphite sheet, a piece of 5 mm x 5 mm square was cut out. The piece was put onto a glass plate (1 cm x 1 cm square), and then four cornets thereof were fixed thereon using a silver paste (DOTITE 550, manufactured by Fujikura Kasei Co., Ltd.) (sample for measurement of electrical property). This measuring sample was put onto a hot plate heated to 150°C, and heated for 2 minutes to be aged. This sample was set into a Hall effect measuring device (RESITEST, manufactured by TOYO Corp.), and then measuring electrodes were fitted to the silver paste moieties. The current value was set to 10 mA, and the voltage was measured through a nano-voltage meter. The sample for measurement of electrical property was set in a cryostat (manufactured by TOYO Corp.) attached with a freezer to be cooled to 5 K. After the temperature reached 5 K, the resistivity of the graphite film was measured at individual temperatures up to 300 K while the measuring temperature mode was set to 1/T (temperature) and the number of the measured temperatures was set to 40. The residual resistivity ratio thereof was calculated by substituting the measured values into the following expression (2):

$$\text{Residual resistivity ratio} = \rho\,300\,\text{K} / \rho\,5\,\text{K} \qquad (2)$$

When the ratio $\rho\,300\,\text{K}/\rho\,5\,\text{K}$ of a film is 1 or more, the film is judged to be a metallic film having a high quality.

[Measurement of Film Thickness]

The thickness of a film was measured, using a contact type thickness meter.

[Measurement of Electro-conductivity]

The electro-conductivity of a sample was measured by the van der Pauw method. This method is a method most suitable for measuring the electro-conductivity (sheet resistance) of a sample in the form of a thin film. Details of this method are described in Experimental Chemical Lecture 9 (fourth version), Electricity/Magnetism (edited by Incorporated Body, The Chemical Society of Japan, and published by Marzen Co., Ltd. (published on June 5, 1991) (p. 170). This method is characterized in that electrodes are fitted to any four points of edge portions of a thin-film sample having any shape, and the resistivity thereof is measurable. When the sample is even in thickness, a precise measurement can be made. In the present invention, a sample cut into a square was used, and silver paste electrodes were fitted to four corners (edges) of the sample to make a measurement. The measurement made use of a resistivity/DC & AC Hall measuring system, ResiTest 8300 manufactured by TOYO Corp. The electro-conductivity of the sample was calculated in accordance with an expression of "electro-conductivity = 1/(a value of resistivity)", using the resultant resistivity.

[Method for Measuring Variation of Graphite Sheet in Thickness]

As represented by the above-mentioned expression (1), the variation V (%) of a graphite sheet in thickness is a value obtained by multiplying the absolute value of the difference between the film thickness Tmax and the arithmetic average value Tave of the film thickness by 100, and then dividing the resultant value by the arithmetic average value Tave of the film thickness.

(Example 1)

[Method for Producing Polyimide Film]

Into a DMF solution in which 3 equivalents of 4,4' -diaminodiphenyl ether (ODA) were dissolved, 4 equivalents of pyromellitic dianhydride (PMDA) were dissolved to synthesize a prepolymer having the acid anhydride at both terminals thereof. Thereafter, one equivalent of p-phenylenediamine (PDA) was dissolved into a solution containing the prepolymer. In this way, a solution containing 18.5% by weight of polyamic acid was obtained.

While this solution was cooled, an imidizing catalyst containing one equivalent of acetic anhydride, one equivalent of isoquinoline, and DMF was added to this solution, these equivalents being each an equivalent relative to the amount of carboxylic groups contained in the polyamic acid. The solution was stirred and then defoamed. The operations from the stirring to the defoaming were performed while the solution was cooled to 0°C. Next, this mixed solution was applied onto an aluminum foil piece to give a predetermined thickness after the solution would be dried.

In a hot wind oven, the mixed solution layer on the aluminum foil piece was dried at 100°C for 60 seconds to prepare a gel film having self-supporting performance. This gel film was peeled off from the aluminum foil piece, and then fixed to a frame. In the hot wind oven, the gel film was further heated step by step at 250°C for 60 seconds and 450°C for 60 seconds to be dried. As a result, a polyimide film was obtained which was a film of 150 mm x 150 mm square and 3.5 $\mu$m thickness.

[Production of Graphite Sheet for Accelerator Beam Sensor (Carbonization)]

The polyimide film of 150 mm x 150 mm size and 3.5 $\mu$m thickness was sandwiched between graphite sheets of 200 mm x 200 mm size. An electrical furnace was used to heat the resultant workpiece up to 1000°C at a heating rate of 2.5°C/minute in a nitrogen atmosphere, and then keep the temperature at 1000°C for 1 hour to carbonize the film.

[Production of Graphite Sheet for Accelerator Beam Sensor (Graphitization)]

The resultant carbonized film of 121 mm x 121 mm size was sandwiched between graphite sheets of 200 mm x 200 mm size, and further this workpiece was sandwiched between square plates of a CIP material of 200 mm x 200 mm size. The workpiece was put into an electrical furnace for graphitization attached with pressing mechanism to be graphitized. The graphitization was performed by heating the workpiece to 3000°C at a rate of 2.5°C/minute in an argon atmosphere, keeping the workpiece at 3000°C for 30 minutes, and then cooling the workpiece naturally. Over 30 minutes after the temperature reached 3000°C, the workpiece was pressed to adjust the pressure in the thickness direction to 0.5 kgf/cm$^2$, and then the pressing was finished.

The resultant graphite sheet was a 135 mm x 135 mm square. The average value of the thicknesses of the sheet at five sites thereof, i.e., the four corners and the center, was 1.1 $\mu$m. The thickness values at the five sites were each in a range of 15% or less from the average value 1.1 $\mu$m. The ratio between the residual resistivity at 300 K and that at 5 K was 2.1, and the electro-conductivity was 22000 S/cm. The graphite sheet had no eyeball-shaped convex portions.

[Production of Electrode for Accelerator Beam Sensor]

The resultant graphite sheet was cut into a size of about 45 mm x about 100 mm. Both ends thereof were bonded with an electro-conductive adhesive to a frame substrate (having a U-shape, in which a frame-region near one side of a 110 cm$^2$ square had been hollowed) to which printed wiring lines are formed. While this state was kept, the resultant was cut and worked by a laser under the following conditions: a wavelength of 532 nm, a spot size of 20 $\mu$m diameter, a peak of 820 mW, and a frequency of 60000 Hz, and a line sweep rate of 1000 mm/minute. In this way, a product (accelerator beam sensor electrode) was produced in which graphite ribbons were formed to be arranged in parallel to each other at regular intervals on the frame substrate. The widths of the ribbons were each 1 mm, the intervals between the ribbons were each 1 mm, the length of each of the ribbons (the length of a region of the ribbon that was stretched in the air) was 70 mm and the number of the ribbons was 20. The same experiment was further made five times to produce electrodes, for a beam sensor, the total number of which was six.

At the time of the laser working, the graphite ribbons were hardly broken or cut away in the middle of the working although the thickness of the ribbons was as very small as 1.1 $\mu$m. Thus, the production of the electrodes for accelerator beam sensors was succeeded with a high yield. Moreover, the variation of the individual ribbons in electrical resistance between their both ends was 10% or less. Thus, the electrical resistances were very even (the graphite sheet in Patent Document 1 was broken or cut away in the middle, to be poor in yield).

DESCRIPTION OF REFERENCE SIGNS

1: Beam halo
2: Front-side secondary electron capturing electrode
3: Electrode for accelerator beam sensor
4: Rear-side secondary electron capturing electrode
5: Accelerator
10: Beam
20: Capturing electrode
22: Frame substrate
23: Printed wiring lines
30: Graphite ribbons
31: Sensor target
32: Frame substrate
33: Printed wiring lines
40: Capturing electrode
42: Frame substrate
43: Printed wiring lines
100: Accelerator beam sensor

**Claims**

1.   A graphite sheet for a beam sensor, having no eyeball-shaped convex portions on a surface of its a-b plane (sheet plane).

2.   A graphite sheet for a beam sensor, having the variation in film thickness of 20% or less.

3.   The graphite sheet according to claim 1 or 2, which is for an accelerator beam sensor.

4.   The graphite sheet according to claim 2 or 3, having no eyeball-shaped convex portions on a surface of its a-b plane (sheet plane).

5.   The graphite sheet according to any one of claims 1 to 4, having a film thickness less than 2.2 $\mu$m.

6.   The graphite sheet according to any one of claims 1 to 5, wherein the ratio between the resistivity at 5 K and that at 300 K (ratio between the residual resistivities) is 1.2 or more.

7.   The graphite sheet according to any one of claims 1 to 6, having an electro-conductivity of 16000 S/cm or more.

8.   The graphite sheet according to any one of claims 1 to 7, which is obtained by using dehydrating agents and one or more selected from tertiary amines to make a film of an aromatic polyimide having a thickness of 100 nm to 7.3 $\mu$m, and sandwiching the resultant aromatic polyimide film between members of one or more species selected from the group consisting of graphite sheets, glassy carbon sheets, graphite plates and glassy carbon plates while pressing to conduct a heat-treatment at a temperature of 2800°C or higher.

9. An electrode for a beam sensor, wherein the graphite sheet according to any one of claims 1 to 8 is cut into the form of ribbons, and these graphite ribbons are arranged at regular intervals on the same single plane.

10. An electrode for a beam sensor, wherein the graphite sheet according to any one of claims 1 to 8 is cut into the form of ribbons, and these graphite ribbons are arranged on the same single plane in a state that either or both of the widths of the graphite ribbons and intervals between the graphite ribbons are varied.

11. The electrode for beam sensor according to claim 9 or 10, wherein the widths of the graphite ribbons are from 100 $\mu$m to 100 mm, the intervals between the graphite ribbons are from 10 $\mu$m to 100 mm, and the lengths of the graphite ribbons are from 10 mm to 800 mm.

12. A beam sensor, comprising the electrode for a beam sensor according to any one of claims 9 to 11 and a pair of secondary electron capturing electrodes,
wherein the secondary electron capturing electrodes are arranged in parallel, respectively, to the front surface and the rear surface of the electrode for a beam sensor, and receive secondary electrons emitted from the electrode.

13. The beam sensor according to claim 12, wherein a plurality of the electrodes for beam sensors are located to arrange individual electrode planes thereof back and forth while the electrode planes are made parallel to each other, and the graphite ribbons on the individual electrode planes are oriented in directions different from each other.

[Fig.1]

[Fig.2]

[Fig.3]

[Fig.4]

[Fig.5]

[Fig.6]

[Fig.7]

[Fig.8]

[Fig.9]

[Fig.10]

## INTERNATIONAL SEARCH REPORT

| International application No. |
| --- |
| PCT/JP2016/071996 |

**A. CLASSIFICATION OF SUBJECT MATTER**

*G01T1/29*(2006.01)i, *C01B31/02*(2006.01)i, *H05H13/00*(2006.01)i, *H05H13/04*
(2006.01)i

According to International Patent Classification (IPC) or to both national classification and IPC

**B. FIELDS SEARCHED**

Minimum documentation searched (classification system followed by classification symbols)
G01T1/29, C01B31/02, H05H13/00, H05H13/04

Documentation searched other than minimum documentation to the extent that such documents are included in the fields searched
Jitsuyo Shinan Koho            1922–1996    Jitsuyo Shinan Toroku Koho    1996–2016
Kokai Jitsuyo Shinan Koho   1971–2016    Toroku Jitsuyo Shinan Koho    1994–2016

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | JP 2007-101367 A  (Inter-University Research Institute Corporation High Energy Accelerator Research Organization),<br>19 April 2007 (19.04.2007),<br>paragraphs [0012] to [0023], [0038], [0044] to [0045], [0058]; fig. 1 to 2<br>(Family: none) | 1-7,9,11-13<br>8,10 |
| Y | JP 2002-308611 A  (Ube Industries, Ltd.),<br>23 October 2002 (23.10.2002),<br>paragraphs [0012] to [0032]<br>(Family: none) | 8 |
| Y | JP 2007-155649 A  (Mitsubishi Electric Corp.),<br>21 June 2007 (21.06.2007),<br>paragraphs [0010], [0015] to [0017]; fig. 3<br>(Family: none) | 10 |

[X] Further documents are listed in the continuation of Box C.        [ ] See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>16 August 2016 (16.08.16) | Date of mailing of the international search report<br>30 August 2016 (30.08.16) |
| --- | --- |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

International application No.

PCT/JP2016/071996

C (Continuation).  DOCUMENTS CONSIDERED TO BE RELEVANT

| Category* | Citation of document, with indication, where appropriate, of the relevant passages | Relevant to claim No. |
|---|---|---|
| A | JP 2011-161056 A  (Toshiba Corp.),<br>25 August 2011 (25.08.2011),<br>paragraph [0031]; fig. 2<br>& US 2012/0305796 A1<br>paragraph [0043]; fig. 2<br>& WO 2011/099449 A1    & CN 102858407 A | 13 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007101367 A
- JP 2002308611 A
- JP 2015151323 A
- JP 2015191754 A

**Non-patent literature cited in the description**

- Experimental Chemical Lecture 9 (fourth version), Electricity/Magnetism. Marzen Co., Ltd, 05 June 1991, 170